# EUROPÄISCHE PATENTSCHRIFT

(11) **EP 0 980 355 B1**
(45) Veröffentlichungstag und Bekanntmachung des Hinweises auf die Patenterteilung: **16.04.2003**
(21) Anmeldenummer: 98929277.6
(22) Anmeldetag: 30.04.1998
(51) Int. Cl.: C07D 201/08

(54) **VERFAHREN ZUR HERSTELLUNG CYCLISCHER LACTAME**
METHOD FOR PRODUCING CYCLIC LACTAMS
PROCEDE DE FABRICATION DE LACTAMES CYCLIQUES

(30) Priorität: 02.05.1997 DE 19718706
(43) Veröffentlichungstag der Anmeldung: 23.02.2000
(73) Patentinhaber: BASF AKTIENGESELLSCHAFT, 67056 Ludwigshafen (DE)
(72) Erfinder: FUCHS, Eberhard, D-67227 Frankenthal (DE); FISCHER, Rolf, D-69121 Heidelberg (DE)
(74) Vertreter: Kinzebach, Werner, Dr.
(86) Internationale Anmeldenummer: EP9802571
(87) Internationale Veröffentlichungsnummer: WO98050355

(56) Entgegenhaltungen:
- EP-A- 0 481 351
- EP-A- 0 659 741
- WO-A-95/14665
- DE-A- 4 443 125
- DE-A- 19 517 823
- US-A- 4 568 736
- US-A- 5 693 793

## Beschreibung

Die vorliegende Erfindung betrifft ein Verfahren zur Herstellung cyclischer Lactame durch Umsetzung eines ω-Aminocarbonsäurenitrils in Gegenwart wenigstens eines Katalysators.

Cyclische Lactame haben eine breite Anwendung als Ausgangsstoffe zur Herstellung von Polyamiden (Nylons) durch ringöffnende Polymerisation gefunden. Das technisch wichtigste Lactam ist dabei das ε-Caprolactam als cyclisches Amid der ε-Aminocapronsäure, das hauptsächlich zur Herstellung von Nylon 6 (Perlon®) verwendet wird. Der wichtigste Verfahrensweg zur Herstellung von ε-Caprolactam ist die Cyclohexanonoxim-Route, wobei Cyclohexanon mit Hydroxylamin zuerst zum Oxim umgesetzt und anschließend einer Beckmann-Umlagerung zum ε-Caprolactam unterzogen wird. Dieser klassische Herstellungsweg ist verbesserungsbedürftig, da er zum einen mehrstufig ist und zum anderen dabei zwingend Sulfate oder andere Nebenprodukte anfallen.

Neuere Verfahren zur Herstellung cyclischer Lactame setzen daher ω-Aminocarbonsäurenitrile als Edukte ein. Dabei erfolgt z. B. die Herstellung von 6-Aminocapronsäurenitril durch selektive einseitige Hydrierung von Adipodintril.

In der US-A-4 628 085 wird die Umsetzung von 6-Aminocapronsäurenitril mit Wasser in der Gasphase an einem speziellen sauren Kieselgel bei 300 °C beschrieben. Durch Verdünnung des Substrats mit Wasser, Ammoniak und Wasserstoff/Stickstoff kann Caprolactam bei quantitativem Umsatz in einer Selektivität über 95 % erhalten werden, aber bereits innerhalb von 150 h findet durch Desaktivierung des Kieselgels ein merklicher Umsatz- und Selektivitätsrückgang statt.

Ein ähnliches Gasphasenverfahren wird auch in der US-A-4 625 023 beschrieben. Hier wird ein hochverdünnter Gasstrom von 6-Aminocapronsäurenitril, Adipodinitril, Ammoniak, Wasser und Trägergas über ein Kieselgel und ein Kupfer/Chrom/Barium-Titanoxid-Katalysatorbett geleitet. Die Caprolactamselektivität beträgt 91 % bei 85 % Umsatz. Auch hierbei besteht das Problem der Katalysatordesaktivierung.

In der US-A-2 245 129 wird die Herstellung linearer Polyamide in einem zweistufigen Verfahren beschrieben. Dabei wird in einer ersten Stufe durch Erhitzen einer 50 %igen wäßrigen Lösung von 6-Aminocapronsäurenitril auf 220 °C für 20 h ein niedermolekulares Zwischenprodukt gebildet, welches in der zweiten Stufe nach Entfernen von Ammoniak und überschüssigem Wasser nachpolymerisiert wird.

In der US-A-2 301 964 wird die nicht-katalysierte Umsetzung von Aminocapronsäurenitril in Form einer wässrigen Lösung zu Caprolactam bei 285 °C beschrieben. Die Ausbeute liegt deutlich unter 80 % und man erhält zudem einen nicht weiter beschriebenen Rückstand.

Die FR-A-2 029 540 beschreibt ein Verfahren zur Cyclisierung von 6-Aminocapronitril zu Caprolactam, wobei als Katalysatoren metallisches Zn oder Cu-Pulver oder Oxide, Hydroxide, Halogenide, Cyanide des Rubidiums, Bleis, Quecksilbers oder der Elemente mit einer Ordnungszahl 21 bis 30 oder 39 bis 48 Verwendung finden.
Die beschriebenen Katalysatoren werden in diskontinuierlich betriebenen Rührautoklaven als Suspensionskatalysatoren eingesetzt. Caprolactam wird dabei in Ausbeuten bis zu 83 % erhalten. Die vollständige Abtrennung der Katalysatoren vom Wertprodukt Caprolactam bereitet jedoch Probleme, da Caprolactam mit den löslichen Bestandteilen der verwendeten Metalle Verbindungen bilden kann oder Feinstpartikel durch mechanisches Rühren entstehen können.

Die US-A-3 485 821 beschreibt die Cyclisierung von in Wasser gelöster 6-Aminocapronsäure bei Temperaturen von 150 - 350 °C zu Caprolactam.

Aus der DE-A-952 442 ist ein Verfahren bekannt, bei dem man durch hydrierende Aminierung von 5-Formylvaleriansäureestern in zwei Stufen Caprolactam neben Aminocapronsäureestern erhält.

In der US-A-3 988 319 wird ein Verfahren zur Cyclisierung von 6-Aminocapronsäure in Methanol oder Ethanol als Lösungsmittel beschrieben. Zur Vermeidung von Nebenreaktionen der 6-Aminocapronsäure muß jedoch die Aminosäure so langsam in Lösung gebracht werden, dass sie nicht als Feststoff vorliegt. Hierfür sind Temperaturen von etwa 170 °C notwendig. Weiterhin darf der Wassergehalt der Lösung nicht über 40 % ansteigen, da sich ansonsten offenkettige Polymere bilden. Das freiwerdende Reaktionswasser muß bei Wiederverwendung des Alkohols abgetrennt werden.

In Ind. Eng. Chem. Process Des. Dev., 17 (1978) 9 - 16 wird beschrieben, dass die Cyclisierung von 6-Aminocapronsäure in Wasser zu Caprolactam zu signifikanten Oligomerenmengen führt, wenn nicht bei Konzentrationen unter 13 % und Temperaturen von rund 300 °C gearbeitet wird.

A. Blade-Font beschreibt in Tetrahedron Lett., 21 (1980) 2443 - 2446 die Cyclisierung von 6-Aminocapronsäure als Suspension in Toluol in Gegenwart von Aluminiumoxid oder Kieselgel unter Auskreisen des Reaktionswassers. Zur vollständigen Desorption des Caprolactams muß der Katalysator mit Methylenchlorid/Methanol gewaschen und Polymere mit Diethylether ausgefällt werden. Die Ausbeute an Caprolactam beträgt nach 20 h Reaktionszeit 82 % an Aluminiumoxid bzw. 75 % an Kieselgel.

In der EP-A-271 815 wird die Cyclisierung von 6-Aminocapronsäureestern zu Caprolactam beschrieben, indem der Ester in einem aromatischen Kohlenwasserstoff gelöst, bei 100 bis 320°C cyclisiert und gleichzeitig der abgespaltene Alkohol abgetrennt wird.

In der EP-A-376,122 wird die Cyclisierung von 6-Aminocapronsäureestern zu Caprolactam beschrieben, indem der Ester in einem aromatischen Kohlenwasserstoff gelöst und unter zusätzlicher Verwendung von Wasser bei Temperaturen von 230 bis 350 °C cyclisiert wird.

Es ist bekannt, Polyamid-6 zu Caprolactam zurückzuspalten. Die Spaltung erfolgt dabei unter der Einwirkung saurer oder basischer Katalysatoren bei erhöhter Temperatur häufig unter Einwirkung von Wasserdampf, also im Niederdruckbereich.

In Chem. Ing. Techn. 45 (1973) 1510 wird die technische Durchführung eines Spaltverfahrens für Nylon 6-Abfälle mit überhitztem Wasserdampf beschrieben, wobei zur Aufarbeitung die Aufkonzentrierung einer Caprolactam/Wasser-Lösung nötig ist.

In der EP-A-209021 wird die Spaltung in einem Aluminiumoxid-Wirbelbett durchgeführt.

Die EP-A 529 470 setzt als Katalysator zur Polyamid-6-Spaltung Kaliumcarbonat zu und führt die Reaktion bei 250 bis 320 °C unter gleichzeitigem Abdestillieren des Caprolactams im Vakuum durch.

Alle diese Verfahren zur Spaltung von Polyamid-6 zu Caprolactam weisen als Nachteile die energetisch sehr aufwendige Abtrennung großer Wassermengen sowie das Entfernen von Katalysatoren wie Phosphorsäuren und der Salze davon, Kaliumcarbonat oder Alkalimetalloxide auf. Im Falle der Gasphasenreaktionen wird das Polymer auf Temperaturen in der Regel im Bereich von 270 bis 400 °C erhitzt und zusammen mit Wasser in einem Wirbelschichtreaktor gespalten. Nebenproduktbildungen und Deaktivierungen durch Verkleben der Katalysatorschüttung sind die Folge.

Die US-A-4 568 736 beschreibt ein Verfahren zur Herstellung von Polyamiden, wobei ω-Aminonitrile mit Wasser in Gegenwart eines phosphorhaltigen Katalysators, wie z. B. Phosphorsäure, phosphorige Säure, hypophosphorige Säure, etc. umgesetzt werden. Die Umsetzung erfolgt in einem zweistufigen Verfahren, wobei in einem ersten Schritt bei Temperaturen zwischen 200 und 300 °C und einem erhöhten Druck zwischen etwa 14 und 56 bar ein Polyamid-Zwischenprodukt von geringem Molekulargewicht gebildet wird, welches in einem zweiten Schritt unter Druckerniedrigung auf Atmosphärendruck oder Drücke unterhalb des Atmosphärendrucks und gleichzeitiger Temperaturerhöhung unter Bildung hochmolekularer Polyamide nachpolymerisiert wird. Im allgemeinen erfolgt dieser zweite Schritt unter Inertgas. Die so erhaltenen Produkte sind im allgemeinen noch phosphorhaltig. Ihre Qualität entspricht nicht der von Produkten, die durch Polymerisation von cyclischen Lactamen hergestellt werden.

Die WO 95/14665 beschreibt ein Verfahren zur Herstellung cyclischer Lactame durch Umsetzung von Aminocarbonsäurenitrilen mit Wasser in flüssiger Phase in einem Festbettreaktor in Gegenwart von heterogenen Katalysatoren, die unter den Reaktionsbedingungen keine löslichen Bestandteile aufweisen. Die Umsetzung erfolgt dabei in Wasser oder in wasserhaltigen Lösungsmittelgemischen. Die Reaktionstemperatur liegt im allgemeinen bei etwa 140 bis 320 °C, bei erhöhten Drücken im Bereich von bis zu 250 bar. Nachteilig an diesem Verfahren ist die Bildung von unerwünschten Nebenprodukten, wie unter den Reaktionsbedingungen nicht spaltbare Oligomere und 6-Aminocapronsäureamid. Bei der Verwendung von alkoholhaltigen Lösungsmittelgemischen kommt es des weiteren immer zur unerwünschten Esterbildung, wie z. B. zur Bildung von 6-Aminocapronsäureethylester.

Die DE-A-44 43 125 beschreibt ein Verfahren zur Herstellung von Caprolactam durch Erhitzen von 6-Aminocapronsäurenitril in Gegenwart von heterogenen Katalysatoren und Wasser unter erhöhtem Druck, wobei man zuerst eine Mischung aus Nitril, Wasser und einem Alkohol in Gegenwart des Katalysators zu einer Mischung I umsetzt, die neben dem erwünschten Caprolactam auch noch Wasser, Alkohol, 6-Aminocapronsäureester, Ammoniak und hochsiedende Verbindungen, wie 6-Aminocapronsäureamid und Oligomere des Caprolactams umfasst. Diese Mischung wird dann einer destillativen Aufarbeitung unterzogen, wobei eine Kopffraktion, Caprolactam und ein Sumpf erhalten werden. Zur Weiterverarbeitung kann die Kopffraktion wiederum in die erste Reaktionsstufe eingespeist werden. Gewünschtenfalls kann sie aber auch gemeinsam mit dem Sumpf in einen weiteren Reaktor eingespeist, gegebenenfalls wiederum mit Alkohol und/oder Wasser und/oder 6-Aminocapronsäurenitril vermischt und anschließend ebenfalls zu Caprolactam umgesetzt und destillativ aufbereitet werden. Gewünschtenfalls kann auch nur der bei der Destillation erhaltene Sumpf in den ersten Reaktor zurückgeführt oder in einem weiteren Reaktor, gegebenenfalls nach Vermischen mit Wasser und/oder einem Alkohol, erneut erhitzt und wiederum zu Caprolactam aufgearbeitet werden. Gewünschtenfalls kann der Sumpf auch nur mit Wasser versetzt und ohne Zusatz eines Katalysators in einem separaten Reaktor erhitzt und anschließend zu Caprolactam aufgearbeitet werden. Gewünschtenfalls kann auch der mit Wasser und einer Base versetzte Sumpf in einem weiteren Reaktor erhitzt und ebenfalls zu Caprolactam aufgearbeitet werden. Um nach diesem Verfahren gute Umsatzraten und Ausbeutung zu erzielen, ist es erforderlich, die bei der Destillation zur Gewinnung des Caprolactams anfallende Kopffraktion und den Sumpf zu recyclen oder, falls erforderlich, separat aufzuarbeiten. Dies bedingt im ersten Fall längere Reaktorbelegzeiten zur Erzielung eines hohen Umsatzes, im zweiten Fall zusätzliche Investitionen für die erforderlichen Reaktoren. Dadurch wird das Verfahren gegenüber anderen wirtschaftlich benachteiligt. Zudem kommt es, wie schon in dem zuvor beschriebenen Verfahren, beim Einsatz von Alkoholen als Lösungsmittel zu einer unerwünschten Bildung von 6-Aminocapronsäureestern.

Der vorliegenden Erfindung liegt die Aufgabe zugrunde, ein verbessertes Verfahren zur Herstellung von cyclischen Lactamen aus ω-Aminocarbonsäurenitrilen zur Verfügung zu stellen. Dabei sollen insbesondere die zuvor beschriebenen Nachteile, die das Verfahren unwirtschaftlich machen, zumindest teilweise vermieden werden. Auch sollen die neuen Verfahrensmaßnahmen möglichst lange Belegzeiten des Reaktors durch Rückführung eines größeren Teils des Reaktionsansatzes oder hohe Investitionskosten durch dessen Aufarbeitung in separaten Reaktoren vermeiden.

Überraschenderweise wurde nun gefunden, dass die Aufgabe gelöst wird, wenn man ω-Aminocarbonsäurenitrile zuerst katalytisch zu Oligomeren umsetzt und diese dann unter Einsatz von überhitztem Wasserdampf zu cyclischen Lactamen spaltet.

Gegenstand der vorliegenden Erfindung ist daher ein Verfahren zur Herstellung cyclischer Lactame der Formel I: 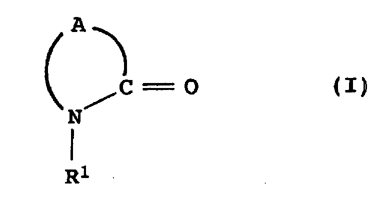 worin
- R¹: für ein Wasserstoffatom, Alkyl, Cycloalkyl oder Aryl steht;
- A: für einen C₃-C₁₂-Alkylenrest steht, der durch 1, 2, 3, 4, 5 oder 6 Substituenten, unabhängig voneinander ausgewählt unter Alkyl, Cycloalkyl oder Aryl, substituiert sein kann;
durch Umsetzung eines ω-Aminocarbonsäurenitrils der Formel II:

HR¹N - A - CN (II)

worin R¹ und A die oben angegebene Bedeutung besitzen, in Gegenwart eines homogenen Katalysators, der eine Phosphorverbindung umfasst, das dadurch gekennzeichnet ist, dass man:
a) das Nitril II zu einem Oligomerengemisch umsetzt,
b) einen Katalysator K1 zugibt und das K1-haltige Oligomerengemisch mit überhitztem Wasserdampf behandelt.

Im Rahmen der vorliegenden Erfindung umfasst der Ausdruck "Alkyl" geradkettige und verzweigte Alkylgruppen. Vorzugsweise handelt es sich dabei um geradkettige oder verzweigte C₁-C₁₂-Alkyl- und insbesondere C₁-C₆-Alkylgruppen. Beispiele für Alkylgruppen sind insbesondere Methyl, Ethyl, Propyl, 1-Methylethyl, Butyl, 1-Methylpropyl, 2-Methylpropyl, 1,1-Dimethylethyl, n-Pentyl, n-Hexyl, n-Heptyl, Octyl, Nonyl, Decyl und Dodecyl.

Cycloalkyl steht vorzugsweise für C₃-C₈-Cycloalkyl, wie Cyclopropyl, Cyclobutyl, Cyclopentyl, Cyclohexyl, Cycloheptyl oder Cyclooctyl, oder Cyclopentylmethyl, Cyclopentylethyl und Cyclohexylmethyl und Cyclohexylethyl.

Aryl steht vorzugsweise für Phenyl, Tolyl oder Naphthyl.

Wird als ω-Aminocarbonsäurenitril der Formel II 6-Aminocapronsäurenitril eingesetzt, so werden als Oligomere z.B. Verbindungen der Formel:

H₂N(̵CH₂)̵₅-CO⁅NH(̵CH₂)̵₅-CO⁆ₘNH(̵CH₂)̵₅-X

worin
m für eine ganze Zahl von 0 bis 20 steht,
X für CN, COOR, CONH₂ oder COOH steht,
R für einen C₁-C₅-Alkylrest steht,
oder Verbindungen der Formel 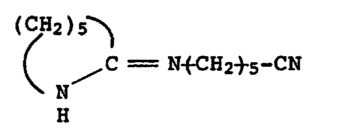 (Caprolactam-iminopentylnitril),
verstanden.

Zusätzlich können im Oligomerengemisch auch Reste an 6-Aminocapronsäurenitril und/oder andere Vorstufen des Caprolactams, wie z.B. 6-Aminocapronsäure und deren Ester und Amide enthalten sein.

Die erfindungsgemäße Herstellung der cyclischen Lactame I erfolgt vorzugsweise kontinuierlich, wobei die Spaltung auch in Batchoder Semibatch-Fahrweise erfolgen kann. Geeignete Reaktoren sind dem Fachmann bekannt und umfassen im allgemeinen Rohrreaktoren, heizbare Rührreaktoren, die für den Verfahrensschritt b) mit einer Vorrichtung zum Zuführen oder Einleiten von überhitztem Wasserdampf und gegebenenfalls mit einer Destillationskolonne versehen sind. Geeignete Reaktionskessel sind in Ullmanns Enzyklopädie der Technischen Chemie, 3. Aufl., Band 1, S. 743 f. beschrieben. Geeignete Reaktionsbehälter für das Arbeiten unter Überdruck finden sich ibid., S. 769 f. Geeignete Destillationskolonnen sind ibid., S. 429 f. beschrieben. Um bei der Herstellung höher siedender cyclischer Lactame im Falle einer destillativen Abtrennung eine Kondensation im Kolonnenkopf zu vermeiden, wird vorzugsweise ein thermostatisierbarer Kolonnenkopf eingesetzt. Gewünschtenfalls können die Schritte a) und/oder b) und/oder eine anschließende Abtrennung des Wassers auch in separaten Reaktoren durchgeführt werden.

### Schritt a)

Nach einer ersten Ausführungsform des erfindungsgemäßen Verfahrens erfolgt die Umsetzung des Nitrils II zu einem Oligomerengemisch in flüssiger Phase in Wasser. Die Temperaturen liegen dabei im allgemeinen in einem Bereich von etwa 100 bis 350 °C, bevorzugt etwa 120 bis 250 °C. Die Reaktionszeit liegt im allgemeinen in einem Bereich von etwa 1 bis 48 Stunden, vorzugsweise etwa 2 bis 24 Stunden.

Das Molmengenverhältnis von Wasser zu ω-Aminocarbonsäurenitril beträgt im allgemeinen etwa 0,01:1 bis 20:1, vorzugsweise etwa 0,5:1 bis 10:1.

Vorteilhafterweise wird das ω-Aminocarbonsäurenitril der Formel II nach dieser Verfahrensvariante lösungsmittelfrei in Wasser eingesetzt. Somit wird die aus dem Stand der Technik bekannte Bildung von entsprechenden ω-Aminocarbonsäureestern vermieden. Gewünschtenfalls können jedoch auch Lösungsmittelgemische aus Wasser und einem inerten Lösungsmittel eingesetzt werden. Geeignete Lösungsmittel sind z. B. aliphatische Kohlenwasserstoffe, wie Petrolether, aromatische Kohlenwasserstoffe, wie Benzol, Toluol und Xylol, Lactame, wie Pyrrolidon, alkylsubstituierte Lactame, wie N-Methylpyrrolidon, N-Methylcaprolactam oder N-Ethylcaprolactam sowie Carbonsäureester, vorzugsweise von Carbonsäuren mit 1 bis 8 C-Atomen.

Vorzugsweise dienen die ω-Aminocarbonsäurenitrile gleichzeitig als Reaktant und Lösungsmittel.

Bei der ersten Verfahrensvariante des Reaktionsschrittes a) wird bereits bei der Umsetzung des Nitrils II zu dem Oligomerengemisch ein Katalysator K1 zugesetzt. Geeignete Katalysatoren K1, die sowohl die Bildung als auch die Spaltung von Oligomeren katalysieren, werden nachfolgend beschrieben. Im allgemeinen kann bei Einsatz eines Katalysators K1 bereits in Schritt a) auf eine weitere Zugabe in Schritt b) verzichtet werden.

Die nach dieser ersten erfindungsgemäßen verfahrensvariante a) erhaltenen Oligomerengemische können im allgemeinen ohne weitere Isolierung oder Aufarbeitung in Reaktionsschritt b) eingesetzt werden. Das bei der Oligomerisierung freiwerdende Ammoniak kann während der Reaktion abgetrennt werden oder im System verbleiben.

Nach einer zweiten Verfahrensvariante können Oligomerengemische entsprechend dem in der US-A-2,245,129 beschriebenen Verfahren hergestellt werden, auf das hiermit Bezug genommen wird. Dabei unterbleibt die dort beschriebene Nachpolymerisation der so erhaltenen Oligomeren. Diese werden stattdessen zur Spaltung in Schritt b) des erfindungsgemäßen Verfahrens eingesetzt.

Nach einer dritten Verfahrensvariante der Stufe a) wird das Nitril II in einem inerten Lösungsmittel und in Abwesenheit von Wasser zu einem Oligomerengemisch umgesetzt. Die Reaktionstemperatur liegt dabei im allgemeinen in einem Bereich von etwa 100 bis 250 °C, bevorzugt etwa 120 bis 230 °C. Die Reaktionszeit liegt im allgemeinen in einem Bereich von etwa 1 bis 80 Stunden, bevorzugt etwa 2 bis 60 Stunden.

Nach einer speziellen Ausführungsform dieser dritten Verfahrensvariante des Reaktionsschrittes a) erfolgt die Umsetzung des Nitrils II zu dem Oligomerengemisch in Gegenwart eines heterogenen Katalysators K2, der wenigstens ein Oxid, Sulfid, Selenid und/oder Telluride von Elementen der 2., 3. oder 4. Hauptgruppe, der 2. bis 6. Nebengruppe, der Lanthaniden, der Aktimiden, ein Zeolith, ein Phosphat, eine Heteropolysäure, einen Ionenaustauscher, und ellischungen davon, umfasst. Dieser ist im allgemeinen verschieden von dem in Schritt b) eingesetzten Katalysator K1. Geeignete Katalysatoren K2 werden nachfolgend beschrieben. Im Anschluss an die Umsetzung wird der Katalysator K2 vom resultierenden Oligomerengemisch abgetrennt, was bei Einsatz eines heterogenen Katalysators durch übliche Verfahren, wie z. B. Sedimentieren, Filtrieren oder Zentrifugieren erfolgt. Geeignete Verfahren und Vorrichtungen zu ihrer Durchführung sind in Ullmanns Enzyklopädie der Technischen Chemie, 3. Aufl., Band 1, S. 470 f. beschrieben.

Nach einer vierten Verfahrensvariante des Reaktionsschritts a) werden Oligomerengemische mit niedervalenten Rutheniumkomplexen, wie von S.-I. Murahashi in Chemtracts: Inorg. Chem. 8 (1996), S. 89-105 beschrieben, oder durch Kupferkatalyse nach üblichen, dem Fachmann bekannten Verfahren, hergestellt.

Vor der Weiterverarbeitung des Oligomerengemisches in Verfahrensschritt b) werden im allgemeinen auch das zugesetzte inerte Lösungsmittel und/oder nicht umgesetztes Nitril II und/oder weitere leichtflüchtige Nebenprodukte aus dem Gemisch entfernt. Dies kann z. B. durch Destillation, vorzugsweise unter verringertem Druck, wie z. B. etwa 1 bis 100 mbar, bei Temperaturen im Bereich der zuvor gewählten Reaktionstemperatur z. B. mit Hilfe der für die Verfahrensstufe b) benötigten Destillationskolonne erfolgen.

Geeignete inerte Lösungsmittel zur Herstellung der Oligomerengemische nach der zweiten Verfahrensvariante von a) sind die zuvor bei der ersten verfahrensvariante genannten inerten Lösungsmittel.

Nach der Abtrennung des Katalysators K2 und/oder weiterer flüchtiger Bestandteile wird das Oligomerengemisch analog zu einem nach der ersten Verfahrensvariante erhaltenen Oligomerengemisch zur Spaltung in Schritt b) eingesetzt.

### Schritt b)

Zur erfindungsgemäßen Herstellung der cyclischen Lactame der Formel I werden die Oligomerengemische in Gegenwart eines Katalysators K1 mit überhitztem Wasserdampf behandelt und dabei gespalten und gegebenenfalls gleichzeitig fraktioniert. Dazu wird dem Oligomerengemisch ein Katalysator K1 zugegeben, sofern dieser nicht bereits aus Schritt a) enthalten ist. Zur Behandlung wird überhitzter Wasserdampf eingesetzt, der im allgemeinen in das Reaktionsgefäß mit dem Oligomerengemisch eingeleitet wird. Die Einleitung in das Reaktionsgefäß erfolgt z. B. über Tauchrohre unterhalb des Flüssigkeitsspiegels des Gemisches. Dabei liegt die Temperatur des Reaktionsgemisches im allgemeinen in einem Bereich von etwa 200 bis 350 °C, bevorzugt etwa 220 bis 300 °C. Die Temperatur des überhitzten Wasserdampfes liegt im allgemeinen in einem Bereich von etwa 240 bis 320 °C, bevorzugt etwa 260 bis 300 °C.

Um bei höher siedenden Lactamen ein Absetzen von festem Produkt im Kolonnenkopf zu vermeiden, kann dieser, wie zuvor erwähnt, thermostatisierbar ausgeführt sein.

Der Durchsatz an Wasserdampf beträgt im allgemeinen etwa 200 bis 800 g/l Ansatz · Stunde, bevorzugt etwa 400 bis 600 g/l Ansatz · Stunde.

Es wird ein wässriges Gemisch oder wässrige Fraktionen von Lactamen der Formel I erhalten, wobei der Gehalt an I mit zunehmender Dauer der Umsetzung bzw. der Fraktionierung abnimmt. Das Ende der Spaltung lässt sich an einer Temperaturabnahme des Destillats am Kolonnenkopf erkennen. Vorzugsweise werden nur produkthaltige Fraktionen mit einem Lactamgehalt von mehr als 5 Gew.-%, bevorzugt mehr als 10 Gew.-% für die anschließende Abtrennung des Wassers eingesetzt.

Der bei der Fraktionierung in Verfahrensschritt b) anfallende Sumpf kann vorzugsweise für eine erneute Spaltung eingesetzt werden.

Im Anschluss an Schritt b) können das Wasser und ggf. noch enthaltene Leichtsieder von den lactamhaltigen Fraktionen abgetrennt werden. Die Abtrennung des Wassers von den lactamhaltigen Fraktionen erfolgt nach üblichen, dem Fachmann bekannten Verfahren.

Dazu zählt z. B. die Destillation bei Normal- oder Unterdruck. Fallen bei der Abtrennung von Leichtsiedern Komponenten an, die als Monomerbausteine geeignet sind, wie z.B. 6-Aminocapronsäurenitril oder 6-Aminocapronsäureester, so können diese erneut zur Oligomerisierung in Verfahrensstufe a) zurückgeführt werden.

Bevorzugt werden nach dem erfindungsgemäßen Verfahren cyclische Lactame hergestellt, die nicht N-substituiert sind. In den Formeln I und II steht R¹ dann für Wasserstoff. Des weiteren bevorzugt werden nach dem erfindungsgemäßen Verfahren cyclische Lactame der Formel I hergestellt, deren Alkylenrest nicht substituiert ist. In den Formel I und II steht A dann für einen unsubstituierten C₃-C₁₂-Alkylenrest.

Besonders bevorzugt steht A für einen C₃-, C₅- oder C₁₁-Alkylenrest. Die entsprechenden cyclischen Lactame der Formel I sind γ-Butyrolactam, ε-Caprolactam und Laurinlactam.

Insbesondere bevorzugt handelt es sich bei dem cyclischen Lactam der Formel I um ε-Caprolactam.

Bei dem erfindungsgemäßen Verfahren handelt es sich bei dem Katalysator K1 um einen homogenen Katalysator, der eine Phosphorverbindung umfasst. Geeignete Katalysatoren sind z. B. die in der US-A-4,568,736 zur Herstellung von Polyamiden beschriebenen Katalysatoren, die gleichzeitig auch die Spaltung von oligomeren Amiden katalysieren. Dazu zählen z. B. Phosphorsäure, Diphosphorsäure, Methaphosphorsäure und Polyphosphorsäuren. Des weiteren geeignet sind die Salze und Ester der phosphorigen Säure, wie die Trialkylphosphite, z. B. Trimethylphosphit und Triethylphosphit, und die Triarylphosphite, wie z. B. Triphenylphosphit. Weitere geeignete Katalysatoren K1 sind die Phosphonsäure, deren organische Derivate mit einer P-C-Bindung, wie z. B. die Alkylphosphonsäuren und Arylphosphonsäuren sowie die Ester und Salze der Phosphonsäure, die Phosphonate und die Ester und Salze der organischen Derivate der Phosphonsäure, die Alkylphosphonate und Arylphosphonate. Weitere geeignete Katalysatoren K1 sind die Ester und Salze der phosphonigen Säure, die Phosphonite. Des weiteren geeignet sind auch die Phosphinsäure sowie deren Ester und Salze, die Phosphinate. Die zuvor genannten Katalysatoren K1 können alleine oder in Mischungen eingesetzt werden.

Nach einer bevorzugten Ausführungsform des erfindungsgemäßen Verfahrens wird als Katalysator K1 ortho-Phosphorsäure oder eine Polyphosphorsäure eingesetzt.

Die Menge des Katalysators K1 beträgt im allgemeinen etwa 0,01 bis 10 Gew.-%, bevorzugt 0,1 bis 3 Gew.-%, bezogen auf die Menge an ω-Aminocarbonsäurenitril der Formel II.

Vorzugsweise wird als Katalysator K2 ein heterogener Katalysator eingesetzt.

Als heterogene Katalysatoren K2 können beispielsweise verwendet werden: Saure, basische oder amphotere Oxide der Elemente der zweiten, dritten oder vierten Hauptgruppe des Periodensystems, wie Calciumoxid, Magnesiumoxid, Boroxid, Aluminiumoxid, Zinn-Oxid oder Siliciumdioxid, wie pyrogen hergestelltes Siliciumdioxid, Kieselgel, Kieselgur, Quarz oder Mischungen derselben, weiterhin Oxide von Metallen der zweiten bis sechsten Nebengruppe des Periodensystems, wie Zirkonoxid, Zinkoxid oder Manganoxid und vorzugsweise Titanoxid (amorph, Anatas oder Rutil), oder Mischungen davon. Ebenfalls verwendbar sind Oxide der Lanthaniden und Aktiniden, wie Ceroxid, Thoriumoxid, Praseodymoxid, Samariumoxid, Seltenerd-Mischoxid, oder Mischungen davon mit zuvor genannten Oxiden. Weitere Katalysatoren können beispielsweise sein: Vanadiumoxid, Nioboxid, Eisenoxid, Chromoxid, Molybdänoxid, Wolframoxid oder Mischungen davon. Mischungen der genannten Oxide untereinander sind ebenfalls möglich. Auch einige Sulfide, Selenide und Telluride, wie Zink-Tellurid, Zinn-Selenid, Molybdänsulfid, Wolframsulfid, Sulfide des Nickels, Zinks und Chroms sind einsetzbar.

Die vorstehend genannten Verbindungen können mit Verbindungen der 1. und 7. Hauptgruppe des Periodensystems dotiert sein bzw. diese enthalten.

Weiterhin können Zeolithe, Phosphate und Heteropolysäuren sowie saure und alkalische Ionenaustauscher wie Naphion® als geeignete Katalysatoren eingesetzt werden.

Gegebenenfalls können diese Katalysatoren bis zu jeweils 50 Gew.-% an Kupfer, Zinn, Zink, Mangan, Eisen, Kobalt, Nickel, Ruthenium, Palladium, Platin, Silber oder Rhodium enthalten.

Die Katalysatoren K2 können je nach der Zusammensetzung des Katalysators als Vollkontakt oder Trägerkatalysator verwendet werden. So kann z.B. Titandioxid in Form von Strängen oder als auf einen Träger in dünner Schicht aufgebrachtes Titandioxid eingesetzt werden. Zum Aufbringen von TiO₂ auf einen Träger wie Siliciumdioxid, Aluminiumoxid oder Zirkondioxid sind alle in der Literatur beschriebenen Methoden verwendbar. So kann eine dünne TiO₂-Schicht durch Hydrolyse von Ti-Organylen wie Ti-Isopropylat oder Ti-Butylat, oder durch Hydrolyse von TiCl₄ oder anderen anorganischen Ti-haltigen Verbindungen aufgebracht werden. Auch Titanoxid-haltige Sole sind verwendbar.

Die Menge des Katalysators K2 beträgt im allgemeinen etwa 0,01 bis 5 Gew.-%, bevorzugt etwa 0,1 bis 3 Gew.-%, bezogen auf die Menge an ω-Aminocarbonsäure.

Die Erfindung wird anhand der folgenden nicht einschränkenden Beispiele näher erläutert.

### Beispiele

### Beispiel 1:

In einem 500 ml Dreihals-Kolben, der mit einer 15 cm Glasrohr-Vakuum-Kolonne ohne Füllung, einem Einleitungsrohr für Wasserdampf und einem elektrischen Thermometer versehen ist, werden 250 g (2,2 mol) 6-Aminocapronsäurenitril mit 5 g Polyphosphorsäure (Dichte 2,6 g/ml; 2 Gew.-% bezogen auf Nitril) gelöst in 20 g (1,1 mol) Wasser, gemischt. Die Beheizung des Kolbens erfolgt mittels eines 800 W Spiegelbrenners. Auf der Kolonne sitzt ein auf 80 °C thermostatisierter Kolonnenkopf mit wassergekühltem Intensivkühler. Die trübe Lösung wird 18 Stunden unter Rückfluß erhitzt, wobei die Temperatur von 133 auf 155 °C ansteigt. Anschließend wird der Ansatz mittels des Spiegelbrenners auf 250 °C erwärmt, wobei 5 g Aminocapronsäurenitril als Destillat entnommen werden. Danach wird mit einer Zuflussrate von 125 g/h auf 275 °C erhitzter Wasserdampf ebenfalls bei 250 °C eingeleitet, wobei der überhitzte Wasserdampf drucklos in einer ölbeheizten Rohrschlange (1 500 mm Länge; 6 mm Durchmesser) auf die gewünschte Temperatur überhitzt und in den Reaktionskolben eingeleitet wird. Der durch das 270 - 275 °C heiße Reaktionsmedium geleitete caprolactamhaltige Wasserdampf wird im Kolonnenkopf bei 80 °C kondensiert. Das Destillat wird in wechselnden Fraktionen aufgefangen: Nach einer Stunde erhält man 201 g einer 33,1 %igen wässrigen Caprolactamlösung. Diese Fraktion enthält außerdem 30,4 g (0,27 mol) Caprolactam-iminopentylnitril (Caprolactim-(6-aminocapronsäurenitril)) und wenig nicht umgesetztes Aminocapronsäurenitril. Nach weiteren zwei Stunden werden 315 g einer 18,7%igen Caprolactamlösung abgenommen, wiederum nach zwei Stunden 277 g einer 14,4 %igen Caprolactamlösung, dann nach 45 Minuten 94 g einer 8,7 %igen Caprolactamlösung und nach weiteren 1,5 Stunden 211 g einer nur mehr 2,7 %igen Caprolactamlösung. Insgesamt erhält man so in 7,3 Stunden 1099 g einer 15,8 %igen (174 g Caprolactam) Caprolactamlösung. Das Ende der Spaltung erkennt man an einer sinkenden Kopftemperatur, der Sumpfanteil von 21,5 g besteht aus Katalysator und Restoligomeren und kann wiederum für eine Spaltung eingesetzt werden.

Die Ausbeute beträgt 76 % bei einem Umsatz von 87 %. Die Selektivität beträgt 87 %. Durch Wiederverwendung des Sumpfanteils erhöht sich die Selektivität an Caprolactam.

### Beispiel 2:

In einer Apparatur nach Beispiel 1 werden 1000 g 6-Aminocapronsäurenitril in 500 g o-Xylol zusammen mit 100 g Titanoxid für 40 Stunden bei 160 °C unter Rückfluß erhitzt. Danach wird die Lösung vom suspendierten Titanoxid abgetrennt und das Lösungsmittel abdestilliert und der Rückstand bei 1 mbar und 156 - 158 °C destilliert. Hierbei erhält man 600 g reines Caprolactam-iminopentylnitril. Im Sumpf verbleiben 75 g nicht umgesetztes Aminocapronsäurenitril und 250 g im wesentlichen spaltbares Polymer.

Entsprechend Beispiel 1 werden 250 g des Caprolactam-iminopentylnitrils mit 5 g Polyphosphorsäure (Dichte 2,6 g/ml; 2 Gew.-% bezogen auf Edukt) versetzt und der Ansatz mittels eines Spiegelbrenners auf 250 °C erwärmt. Bei 250 °C werden mit einer Zuflussrate von 125 g/h auf 275 °C erhitzter Wasserdampf eingeleitet. Der durch das 270 - 275 °C heiße Reaktionsmedium geleitete caprolactamhaltige Wasserdampf wird im Kolonnenkopf bei 80 °C kondensiert und das Destillat in wechselnden Fraktionen aufgefangen. Nach 1 Stunde erhält man 260 g einer 33,8 %igen wässrigen Caprolactamlösung. Diese Fraktion enthält außerdem 78 g (0,7 mol) Caprolactam-iminopentylnitril und Spuren nicht umgesetztes Aminocapronsäurenitril. Nach einer weiteren Stunde werden 146 g einer 20,2 %igen Caprolactamlösung abgenommen, nach weiteren 2 Stunden 300 g einer 14,4 %igen Caprolactamlösung und wiederum nach zwei Stunden 258 g einer 7,9 %igen Caprolactamlösung. Insgesamt erhält man so im Verlauf von 6 Stunden 964 g einer 18,8 %igen (181 g Caprolactam) Caprolactamlösung. Das Ende der Spaltung erkennt man an einer sinkenden Kopftemperatur. Der verbleibende Sumpfanteil von 15,5 % besteht aus Katalysator und Restoligomeren und kann wiederum für eine Spaltung eingesetzt werden.

Die Ausbeute beträgt 67 % bei einem Umsatz von 71 %. Die Selektivität beträgt 95 %. Durch Wiederverwendung des Sumpfanteils erhöht sich die Selektivität an Caprolactam.

## Patentansprüche

1. Verfahren zur Herstellung cyclischer Lactame der Formel I: 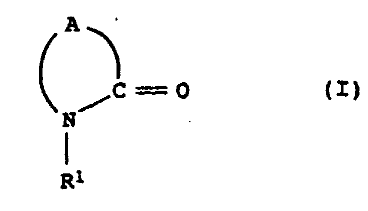 worin
R¹ für ein Wasserstoffatom, Alkyl, Cycloalkyl oder Aryl steht;
A für einen C₃-C₁₂-Alkylenrest steht, der durch 1, 2, 3, 4, 5 oder 6 Substituenten, unabhängig voneinander ausgewählt unter Alkyl, Cycloalkyl oder Aryl, substituiert sein kann;
durch Umsetzung eines ω-Aminocarbonsäurenitrils der Formel II:
HR¹N - A - CN (II)
worin R¹ und A die oben angegebene Bedeutung besitzen, in Gegenwart eines homogenen Katalysators, der eine Phosphorverbindung umfasst, **dadurch gekennzeichnet, dass** man:
a) das Nitril II zu einem Oligomerengemisch umsetzt,
b) einen Katalysator K1 zugibt und das K1-haltige Oligomerengemisch mit überhitztem Wasserdampf behandelt.

2. Verfahren nach Anspruch 1, **dadurch gekennzeichnet, dass** die Umsetzung des Nitrils II in Stufe a) in Gegenwart von Wasser erfolgt.

3. Verfahren nach Anspruch 2, **dadurch gekennzeichnet, dass** auch die Umsetzung in Stufe a) in Gegenwart des Katalysators K1 erfolgt.

4. Verfahren nach Anspruch 1, **dadurch gekennzeichnet, dass** die Umsetzung des Nitrils II in Stufe a) in einem inerten Lösungsmittel erfolgt.

5. Verfahren nach Anspruch 4, **dadurch gekennzeichnet, dass** die Umsetzung in Stufe a) in Gegenwart eines heterogenen Katalysators K2, der wenigstens ein Oxid, Sulfid, Selenid und/oder Tellurid von Elementen der 2., 3. oder 4. Hauptgruppe, der 2. bis 6. Nebengruppe, der Lanthaniden, der Aktiniden, ein Zeolith, ein Phosphat, eine Heteropolysäure, einen Ionenaustauscher, und Mischungen davon, umfasst, erfolgt.

6. Verfahren nach einem der Ansprüche 4 oder 5, **dadurch gekennzeichnet, dass** nach der Umsetzung in Stufe a) der Katalysator K2 sowie gegebenenfalls das Lösungsmittel und/oder nicht umgesetztes Nitril und/oder weitere Nebenprodukte aus dem Oligomerengemisch entfernt werden.

7. Verfahren nach einem der vorhergehenden Ansprüche, **dadurch gekennzeichnet, dass** die Umsetzung in Stufe a) bei Temperaturen im Bereich von 100 bis 350 °C, bevorzugt 120 bis 250 °C, erfolgt.

8. Verfahren nach einem der vorhergehenden Ansprüche, **dadurch gekennzeichnet, dass** die Behandlung des Oligomerengemisches in der Stufe b) bei Temperaturen im Bereich von 200 bis 350 °C, bevorzugt 220 bis 300 °C, erfolgt.

9. Verfahren nach einem der vorhergehenden Ansprüche, **dadurch gekennzeichnet, dass** man in Stufe b) den überhitzten Wasserdampf in das Oligomerengemisch einleitet.

10. Verfahren nach einem der vorhergehenden Ansprüche, **dadurch gekennzeichnet, dass** die Menge des Katalysators K1 0,01 bis 10 Gew.-%, bevorzugt 0,1 bis 3 Gew.-%, bezogen auf die Menge an ω-Aminocarbonsäurenitril beträgt.

11. Verfahren nach einem der Ansprüche 5 bis 10, **dadurch gekennzeichnet, dass** man einen Katalysator K2 einsetzt, der zusätzlich wenigstens eine Verbindung der 1. und/oder 2. Hauptgruppe des Periodensystems umfasst.

12. Verfahren nach einem der Ansprüche 5 bis 11, **dadurch gekennzeichnet, dass** man einen Katalysator K2 einsetzt, der wenigstens ein Metall, ausgewählt unter Ti, Cu, Sn, Zn, Mn, Fe, Co, Mi, Ru, Pd, Pt, Ag, Rh, umfasst.

13. Verfahren nach einem der Ansprüche 5 bis 12, **dadurch gekennzeichnet, dass** die Menge des Katalysators K2 0,01 bis 5 Gew.-%, bevorzugt 0,1 bis 3 Gew.-%, bezogen auf die Menge an ω-Aminocarbonsäurenitril beträgt.

14. Verfahren nach einem der vorhergehenden Ansprüche, **dadurch gekennzeichnet, dass** es sich bei dem Lactam der Formel I um ε-Caprolactam handelt.

## Claims

1. A process for preparing cyclic lactams of the formula I: 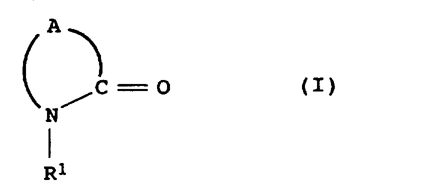 where
R¹ is hydrogen, alkyl, cycloalkyl or aryl, and
A is C₃-C₁₂-alkylene, unsubstituted or substituted by 1, 2, 3, 4, 5 or 6 substituents selected independently of one another from the group consisting of alkyl, cycloalkyl and aryl,
by conversion of an ω-aminocarbonitrile of the formula II:
HR¹N - A - CN (II)
where R¹ and A are each as defined above, in the presence of a homogeneous catalyst comprising a phosphorus compound, which process comprises:
a) converting said nitrile II into an oligomer mixture,
b) adding a catalyst K1 and treating said K1-comprising oligomer mixture with superheated steam.

2. A process as claimed in claim 1, wherein said converting of said nitrile II in step a) is effected in the presence of water.

3. A process as claimed in claim 2, wherein said converting in step a) is likewise effected in the presence of said catalyst K1.

4. A process as claimed in claim 1, wherein said converting of said nitrile II in step a) is effected in an inert solvent.

5. A process as claimed in claim 4, wherein said converting in step a) is effected in the presence of a heterogeneous catalyst K2 which comprises at least one oxide, sulfide, selenide and/or telluride of elements of the 2nd, 3rd or 4th main group, of the 2nd, 3rd, 4th, 5th or 6th transition group, of the lanthanides, of the actinides, a zeolite, a phosphate, a heteropolyacid, an ion exchanger and mixtures thereof.

6. A process as claimed in either of claims 4 and 5, further comprising removing said catalyst K2 and, if appropriate, said solvent and/or unconverted nitrile and/or further by-products from said oligomer mixture after said converting in step a).

7. A process as claimed in any of the preceding claims, wherein said converting in step a) is effected at from 100 to 350°C, preferably at from 120 to 250°C.

8. A process as claimed in any of the preceding claims, wherein said treating of said oligomer mixture in said step b) is effected at from 200 to 350°C, preferably at from 220 to 300°C.

9. A process as claimed in any of the preceding claims, wherein said superheated steam is passed into said oligomer mixture in step b).

10. A process as claimed in any of the preceding claims, wherein said catalyst K1 is used in an amount within the range from 0.01 to 10% by weight, preferably within the range from 0.1 to 3% by weight, based on ω-aminocarbonitrile.

11. A process as claimed in any of claims 5 to 10, wherein said catalyst K2 further comprises at least one compound of the 1st and/or 2nd main group(s) of the periodic table.

12. A process as claimed in any of claims 5 to 11, wherein said catalyst K2 comprises at least one metal selected from the group consisting of Ti, Cu, Sn, Zn, Mn, Fe, Co, Ni, Ru, Pd, Pt, Ag and Rh.

13. A process as claimed in any of claims 5 to 12, wherein said catalyst K2 is used in an amount within the range from 0.01 to 5% by weight, preferably within the range from 0.1 to 3% by weight, based on ω-aminocarbonitrile.

14. A process as claimed in any of the preceding claims, wherein said lactam of said formula I is ε-caprolactam.

## Revendications

1. Procédé de préparation de lactames cycliques de la formule I : 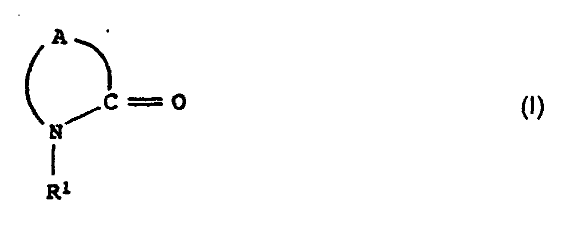 dans laquelle
R¹ représente un atome d'hydrogène ou un groupe alkyle, cycloalkyle ou aryle,
A représente un radical alkylène en C₃-C₁₂ qui peut être substitué par 1, 2, 3, 4, 5 ou 6 substituants, choisis indépendamment l'un de l'autre parmi de l'alkyle, du cycloalkyle ou de l'aryle,
par réaction d'un ω-aminocarbonitrile de la formule II :
HR¹N - A - CN (II)
dans laquelle R¹ et A possèdent la signification indiquée ci-dessus, en présence d'un catalyseur homogène qui comporte un composé de phosphore, **caractérisé en ce que** :
a) on transforme le nitrile II en un mélange d'oligomères,
b) on ajoute un catalyseur K1 et on traite le mélange d'oligomères contenant K1 par de la vapeur d'eau surchauffée.

2. Procédé suivant la revendication 1, **caractérisé en ce que** la conversion du nitrile II dans l'étape a) a lieu en présence d'eau.

3. Procédé suivant la revendication 2, **caractérisé en ce que** la conversion dans l'étape a) a également lieu en présence du catalyseur K1.

4. Procédé suivant la revendication 1, **caractérisé en ce que** la conversion du nitrile II dans l'étape a) a lieu dans un solvant inerte.

5. Procédé suivant la revendication 4, **caractérisé en ce que** la conversion dans l'étape a) a lieu en présence d'un catalyseur hétérogène K2, qui comporte au moins un oxyde, sulfure, séléniure et/ou tellurure d'éléments des groupes principaux 2, 3 ou 4, des groupes secondaires 2 à 6, des lanthanides, des actinides, une zéolite, un phosphate, un hétéropolyacide, un échangeur d'ions, et des mélanges de ceux-ci.

6. Procédé suivant une des revendications 4 et 5, **caractérisé en ce que**, après la conversion dans l'étape a), on élimine du mélange d'oligomères le catalyseur K2 ainsi qu'éventuellement le solvant et/ou du nitrile n'ayant pas réagi et/ou d'autres produits secondaires.

7. Procédé suivant l'une des revendications précédentes, **caractérisé en ce que** la conversion dans l'étape a) a lieu à des températures de l'ordre de 100 à 350°C, de préférence de 120 à 250°C.

8. Procédé suivant l'une des revendications précédentes, **caractérisé en ce que** le traitement du mélange d'oligomères dans l'étape b) a lieu à des températures de l'ordre de 200 à 350°C, de préférence de 220 à 300°C.

9. Procédé suivant l'une des revendications précédentes, **caractérisé en ce que**, dans l'étape b), on introduit la vapeur d'eau surchauffée dans le mélange d'oligomères.

10. Procédé suivant l'une des revendications précédentes, **caractérisé en ce que** la quantité du catalyseur K1 est de 0,01 à 10% en poids, de préférence de 0,1 à 3% en poids, par rapport à la quantité de l'ω-aminocarbonitrile.

11. Procédé suivant l'une des revendications 5 à 10, **caractérisé en ce qu'**on met en oeuvre un catalyseur K2 qui comporte en supplément au moins un composé des groupes principaux 1 et/ou 2 du système périodique.

12. Procédé suivant l'une des revendications 5 à 11, **caractérisé en ce qu'**on met en oeuvre un catalyseur K2, qui comporte au moins un métal choisi parmi Ti, Cu, Sn, Zn, Mn, Fe, Co, Ni, Ru, Pd, Pt, Ag, Rh.

13. Procédé suivant l'une des revendications 5 à 12, **caractérisé en ce que** la quantité du catalyseur K2 est de 0,01 à 5% en poids, de préférence de 0,1 à 3% en poids, par rapport à la quantité de l'ω-aminocarbonitrile.

14. Procédé suivant l'une des revendications précédentes, **caractérisé en ce que**, pour ce qui concerne le lactame de la formule I, il s'agit d'ε-caprolactame.
